# EUROPEAN PATENT SPECIFICATION

(11) **EP 2 032 057 B1**
(45) Date of publication and mention of the grant of the patent: **09.12.2009**
(21) Application number: 07764489.6
(22) Date of filing: 12.06.2007
(51) Int. Cl.: A61B 18/14, A61N 1/32

(54) **ELECTRODE INTRODUCER DEVICE**
ELEKTRODENEINFÜHREINRICHTUNG
DISPOSITIF D'INTRODUCTION D'ÉLECTRODES

(30) Priority: 12.06.2006 DK 200600788; 12.06.2006 US 812601 P
(43) Date of publication of application: 11.03.2009
(73) Proprietor: Region Hovedstaden v/Herlev Hospital, 2730 Herlev (DK)
(72) Inventor: GEHL, Karen Julie, DK-2720 Vanlose (DK); VIDEBÆK, Karsten, DK- 4040 Jyllinge (DK)
(74) Representative: Plougmann & Vingtoft A/S
(86) International application number: PCT/DK2007/050069
(87) International publication number: WO 2007/144004

(56) References cited:
- US-A1- 2003 212 394
- US-A1- 2004 059 328

## Description

The present invention concerns a device for electroporation, in general and more specifically the present invention concerns a device for administering therapeutical molecules, such as a drug, an isotope or genetic material enhanced by electric pulses causing electroporation of and/or electrophoretic effects in a target region of a patient's body.

### BACKGROUND OF THE INVENTION

In the treatment of diseases in the brain, e.g. brain cancer, as well as diseases in other anatomical areas of a body, physical access to a diseased tissue region may be a challenge. This is especially the case if the diseased region lies deep within the body of the patient. Furthermore, efficient delivery and subsequent uptake of therapeutic molecules, such as a drug or genetic compound, to an anatomical target tissue is often a problem.

Electroporation is a known method used to deliver drugs and genetic material to various biologic tissues, where the uptake of these substances into tissue cells is enhanced through the application of electric pulses of specific amplitude. The delivery of drugs by electroporation is also known as electro-chemotherapy (ECT) and the delivery of genes as Electro Gene Transfer (EGT). In ECT and EGT applications, electroporation is used to create a transient permeabilization of the cell membranes in a target tissue area with the purpose of enhancing the uptake of the chemotherapeutic agents as well as the uptake and expression of genetic materials.

In addition to the delivery of therapeutic molecules, electroporation has a stand-alone application that is known as irreversible electroporation (IRE). In IRE, the amplitude of electric pulses is increased beyond the levels used in ECT and EGT, which creates a permanent permeabilization of the cell membranes in a target tissue area with the purpose of promoting cell death through cell leakage.

In order to provide an efficient electroporation two or more electrode poles have to be brought into - or into close vicinity of - the region to be treated (target region). Examples of devices used for Electroporation are known from US 5 674 267 and US 6 278 895. These devices consist of an array of needle-type electrodes arranged as individual electrodes inserted via some external plate-shaped element providing a fixed distance between and relative position of the individual needles. If the target region is situated in a remote region of the body, such as the deeper regions of the brain, the placement of electrodes may in itself be harmful to intervening tissue through which the electrodes need to traverse in order to be located in the desired region. Furthermore, a large access area must be available, and for applications in the brain this will entail creating a large hole in the patient's skull. Therefore, it is evident that the mentioned prior art devices are only well-suited for treatment in target regions in close proximity to an outer surface of the body, because an attempt to treat deeper-lying regions would cause excessive trauma to the intervening tissue.

### OBJECT OF THE INVENTION

There is thus a need for an electroporation device and an electroporation method that overcomes the shortcomings of the presently known devices and methods. It is an object of the present invention to provide such a device. It is a further object of the invention to provide an electroporation device which can be manoeuvred to deeper-lying regions of the body or to regions that are otherwise difficult to access, and to do so with the least amount of injury to the tissue. E.g. for applications in the brain, it is an objective to provide a device necessitating the smallest possible entry hole while providing the largest possible electric field. A further object of the invention is to provide an electroporation device capable of delivering an improved, flexible and more efficient electric field in order to enhance the transfer of e.g. a drug, isotopes, genetic materials or other therapeutical molecules through cell membranes of a target tissue/region. By providing an improved, more efficient and more readily controlled electrical field, the energy applied through electrodes to the tissue may be reduced. Thereby, unintended damages to the tissue, especially the tissue immediately surrounding the electrodes may be reduced. There is furthermore a need for a device constituting an alternative to the known devices.

### SUMMARY OF THE INVENTION

These and other objectives of the invention are obtained by an electroporation device comprising a handle section; an elongate introducer shaft connected to said handle section, said introducer shaft having a distal tip; and a set of electrodes having respective distal ends, each electrode being slidably arranged within said introducer shaft and said tip from a retracted position, where said distal ends are enclosed within said introducer shaft, to an exposed position, where said distal ends extend from said distal tip; wherein said electrode distal ends are deflectable away from a longitudinal axis of said shaft when deployed/extended to their extended position, such that at least one planar projection taken in a plane perpendicular to said longitudinal axis of a distance between a pair of distal ends of said electrodes is larger than a maximal extent of a cross-section of said introducer shaft, said cross-section taken in a plane perpendicular to said longitudinal axis L at a distal end of said introducer shaft, and further as stated in claim 1 of the accompanying claims.

A significant advantage of the presently described device is that it allows insertion of multiple electrodes to a sub-surface tissue region or target region of a patient's body while causing minimal tissue displacement and damage to intervening tissue. This advantageous effect is obtained due to the small outer extent/diameter or profile of the invasive portion, i.e. the shaft or at least the distal part thereof, of the present device in a direction transversal to the direction of insertion.

The device according to the invention allows sub-surface generation of a pattern of electrode end points (distal ends) resembling e.g. a planar or spatial ellipse or ellipsoidal shape or any other regular or irregular spatial geometric shape that will provide an efficient electric field of controllable shape, suitable for the varying anatomical/geometric shapes of the target regions found in real patients. Furthermore, this can be obtained under such sub-surface circumstances (deeper-lying / difficultly accessible regions) as is not possible with prior art devices, through the sub-surface deployment of multiple electrodes that are angled away from the introducer shaft and comprise respective un-insulated end-points, preferably placed in an equidistant relationship, that may circumscribe the outer periphery of such an ellipsoid or other geometric shape in the target region.

In an embodiment of the electroporation device, the deflection of said distal ends of said electrodes, when in their extended position, is provided by a curving of distributor channels provided in said distal tip. Alternatively or additionally, the deflection of said distal ends of said electrodes, when in their extended position, is provided by tension characteristics of at least a section (in an elongate direction) of said electrodes, i.e. by a biasing of said electrode or electrode section. Alternatively or additionally said electrodes are formed in a material comprising a shape memory alloy.

In one embodiment the distal tip may alternatively or additionally be formed with a substantially smooth, rounded, non-cutting shape with a substantially smooth, non-cutting transition to the introducer shaft proper. Thus, the device has no sharp edges, and injuries to the tissue can be minimized.

In an embodiment the distal tip is connectable to said introducer shaft. Alternatively the tip is formed integrally with the shaft.

In yet another embodiment each of said electrode distal ends can be advanced individually to their extended positions. Thereby, the extended distribution of the electrodes, and thus the shape of the electrical field, may be adapted to the individual target tissue. Alternatively, the electrodes may be advanced or extended from the tip in subsets of electrodes or as one set of electrodes, e.g. such that the length of the individual electrodes are adapted to the target tissue shape.

In yet another embodiment the electrodes are extendable such that said distal ends are extendable to form a spatial distribution around a volume of target tissue/a target region. In one embodiment thereof, the distal ends are extendable to form a substantially spherical distribution pattern. Alternatively at least a subset of said distal ends is extendable to form an ellipsoid pattern in a plane parallel to said longitudinal axis of the introducer shaft.

In any of the above mentioned embodiments said electrodes may be slideably arranged in electrically insulated guide channels formed in the shaft and tip. Alternatively or additionally, said electrodes may be provided with an electric insulation coating, the distal-most part of the electrode distal ends being un-insulated to form point electrodes.

The sub-surface generation of an electric field having a geometric shape resembling an ellipse or other three-dimensional shape will provide a more homogeneous tissue coverage. The subsequent application of short and intense electric pulses to two or more of these, preferably equidistant, electrodes will result in a potential difference between the positive and the negative electrodes and a resulting electric field will be generated between these two or more electrodes.

According to an advantageous embodiment of the invention said introducer shaft further comprises a delivery channel through which a dose of therapeutical molecules can be administered, said delivery channel extending through the length of said shaft and terminating through said distal tip. The delivery channel is provided through the shaft along an elongate axis thereof in order to accommodate the delivery of a dose of therapeutical molecules to the region in the vicinity of the tip of the shaft when the device is inserted into the region of a target tissue. Thus, local administration of therapeutic molecules to a target region can be enhanced. However, it is understood that the device may also be applied in combination with systemic administration of therapeutical molecules, where the electroporation will enhance local uptake of therapeutic molecules in tissue cells in the region/vicinity of the electrodes.

In an embodiment said delivery channel is connectable to an external therapeutic molecule delivery system comprising a therapeutic molecule reservoir and pumping means for administering said therapeutic molecules through said delivery channel. Alternatively, the handle part comprises a therapeutic molecule delivery system comprising a therapeutic molecule reservoir and pumping means for administering said therapeutic molecules through said delivery channel. In either embodiment of the device comprising a delivery channel said device may further be adapted to for introducing e.g. a surgical tool or an ultrasound probe through said delivery channel. This adaptation may comprise a suitable sizing of the delivery channel and/or suitable connection means between the device and the surgical tool. In an alternative embodiment a separate channel may be provided in the shaft for the insertion of a surgical tool or an ultrasound probe.

In one embodiment said introducer shaft has a circular cross section with an outer diameter of 15 mm or less, preferably of 10 mm or less, more preferably of 5 mm or less. However, other cross sections may be provided as well, e.g. oval. in this case the above mention dimensions may apply to the maximal extent across the cross section.

In yet another embodiment the introducer shaft may comprise an outer tube and an inner electrode assembly guide received in said outer tube, and where said electrodes are slideably arranged in electrode guide channels formed in said inner electrode assembly guide. In an embodiment said electrode guide channels are formed in a set of cylindrical guide sheaths that are received in semi-open channels distributed in a longitudinal direction along the periphery of said inner electrode assembly guide. In another embodiment, the introducer shaft may be provided by a multitude of electrode guide channel tubes, each configured to receive one or more electrodes.

The introducer shaft may in one embodiment be rigid. However, in other embodiments the introducer shaft may be flexible and/or steerable, the latter embodiment especially being suitable for insertion through body cavities / transvenous applications.

Such flexible and/or steerable applications may be endoscopic or catheter-based applications in natural channels or lumens in the body, where a flexible/steerable shaft 10 is desirable. Introduction of the device may be through natural anatomical openings or through suitable entry sites such as for instance the femoral artery. Alternatively or additionally, laparoscopic applications through an opening in the abdominal cavity or other areas of the anatomy are envisioned, where the shaft may be introduced via an introducer such as a laparoscope with a working channel or a similar introducer sheath. Such an introducer may, e.g. have a cutting edge, or a removable trocar with a trocar tip that may be removed after insertion of the introducer to facilitate insertion.

In an embodiment, the shaft is substantially straight. However, in other embodiments the shaft may be curved, in order to provide the opportunity to be used in particular anatomic regions (e.g. for tumours of the head and neck) or to circumvent e.g. fragile tissue regions.

Preferably the device comprises 10 or more electrodes. Thus, the electrodes, in extended position, may be spatially distributed to enhance the formation of a spatial electrical field The device, in yet other embodiments comprises 12, 16 or more electrodes.

The device, in yet another embodiment comprises 32 electrodes. In one particular embodiment, said electrodes are slideably arranged within guide channels distributed in groups of four in each of eight cylindrical guide sheaths.

In respect to all of the previously mentioned embodiments an electric stimulus generator may be integrated into the handle section of the device. Alternatively, the device comprises means for connecting/attaching the device electrodes to an external electric stimulus generator.

In one embodiment each electrode or group of electrodes is individually assignable to pass an electrical current, such that the emission of electric stimuli can be provided from individual electrodes or groups of electrodes. Thus an enhanced control of an induced electrical field may be provided. The control or assignment of the individual electrodes or groups of electrodes may be provided by a suitable electronic control unit provided either in the handle part of the device, in the external electronic stimulus generator or as a stand-alone unit.

The tip of the introducer shaft may be a rounded, smooth, atraumatic tip adapted for spreading tissue, thus causing minimal damage to the tissue through which it is to be moved. This is advantageous especially for intravenous applications or applications in e.g. the brain. However, the tip may in other embodiments be provided with a sharpened or cutting tip or a pointed tip that is e.g. suitable for percutaneous applications.

There is further described an electroporation method comprising the steps of providing an electroporation device, the device comprising an elongate introducer shaft having a distal tip; and a set of electrodes having respective distal ends, each electrode being slidably arranged within said introducer shaft from a retracted position, where said distal ends are enclosed within said introducer shaft, to an extended position, where said distal ends extend from said distal tip; the method further comprising the steps of inserting said introducer shaft through tissues of a body and bring said distal tip into a vicinity of a target region of tissue to be treated, while said electrodes are in said retracted position; extending said electrodes to said extended position, such that said electrode distal ends are deflected away from a longitudinal axis of said shaft in such a way that at least one planar projection taken in a plane perpendicular to said longitudinal axis of a distance between a pair of distal ends of said electrodes is larger than a maximal extent of a cross-section of said introducer shaft, said cross-section taken in a plane perpendicular to said a longitudinal axis at a distal end of said introducer shaft; administering a dose of therapeutic molecules to said body; and applying through said electrodes one or more electric pulses, e.g. in a specific sequence to the target region tissue to create a transient permeabilization of cell membranes of tissue in said target region.

In one variant of the electroporation method said dose of therapeutic molecules is administered systemically. In another variant of the electroporation method said dose is administered locally in the vicinity of the target region. Such local administration may advantageously be delivered before, during or after extending said electrodes, through a delivery channel extending through the length of said shaft and terminating through said distal tip. Alternatively said dose may be delivered locally through a suitable addition injection/infusion device.

A further aspect of the method may be the step of inserting into the vicinity of the target region a set of electrodes, having respective distal ends, enclosed (in a first retracted position) within a single elongate introducer shaft having a distal tip; extending at least a pair of said electrodes to a position extended from their position within said shaft, such that said electrode distal ends are deflected away from a longitudinal axis of said shaft in such a way that at least one planar projection taken in a plane perpendicular to said longitudinal axis of a distance between a pair of distal ends of said electrodes is larger than a maximal extent of a cross-section of said introducer shaft, said cross-section taken in a plane perpendicular to said a longitudinal axis at a distal end of said introducer shaft; and applying through said electrodes one or more electric pulses to the target tissue.

In the method as described the electrodes are point electrodes which are positioned such that when a sequence of electric pulses is applied through some or all of said electrodes an ellipsoid or spatially ellipsoid electric field is generated between some of or all the points that are positioned in the tissue. In a further variant said ellipsoid or spatial ellipsoid field is generated by positioning said point electrodes in an ellipsoid or spatially ellipsoid configuration at least partly surrounding or enclosing said target tissue. In yet another variant said point electrode distal ends are positioned in substantially circular parallel layers, where the position of the point electrodes in a section perpendicular to said circular layers defines an ellipsoid configuration. In a further variant the electric field is generated in the tissue by applying a sequence of electric pulses between at least sixteen point electrodes in at least four essentially parallel, consecutive layers comprising at least four point electrodes in each layer and such that said sequence comprises the steps of generating at least some pulses travelling from at least one of the electrodes in first positive layer of point electrodes to at least one of the electrodes in a first negative layer of point electrodes placed in equidistant relation to the electrodes in the first layer, while other pulses simultaneously travel from at least one of the electrodes in a second positive layer to at least one of the electrodes in a second layer of point electrodes, respectfully.

The method of generating an electric field in a target tissue of a patient, may further is comprise the steps of inserting into the vicinity of a target tissue a set of point electrodes, having respective electrically conductive distal ends, and positioning said electrode distal ends in a spatial formation surrounding or enclosing at least partly said target tissue; applying through said point electrodes a sequence of electric pulses to the target tissue.

In a variant of this other the method of generating an electric field in a target tissue of a patient, the point electrodes are positioned such that when a sequence of electric pulses is applied through said electrodes an ellipsoid or spatially ellipsoid electric field is generated in the tissue. In a further variant said ellipsoid or spatial ellipsoid field is generated by positioning said point electrodes in an ellipsoid or spatially ellipsoid configuration at least partly surrounding or enclosing said target tissue. In yet variant said point electrode distal ends are positioned in substantially circular parallel layers and where the position of the point electrodes in a section perpendicular to said circular layers defines an ellipsoid configuration. In a further variant electric field is generated in the tissue by applying a sequence of electric pulses between at least sixteen point electrodes in at least four essentially parallel, consecutive layers comprising at least four point electrodes in each layer and such that said sequence comprises the steps of generating at least some pulses travelling from at least one of the electrodes in a first positive layer of point electrodes to at least one of the electrodes in a first negative layer of point electrodes placed in equidistant relation to the electrodes in the first layer, while other pulses simultaneously travel from at least one of the electrodes in a second positive layer to at least one of the electrodes in a second negative layer of point electrodes, respectively.

The invention is advantageously applied in electro-chemotherapy, electro gene therapy especially for treatment of brain cancers, and other diseases of the brain. The present disclosure describes the invention from this point of view, but it is understood that the device according to the invention may also be adapted for applications in the treatment of diseases of e.g. the liver, lung, kidney or other soft or hard tissues. The invention may further be applied in the field of irreversible electroporation.

The device and method may be applied to treatment of humans as well as animals.

In the present document the term shaft should be taken to mean an elongate structure that is either rigid or flexible/bendable/steerable, and either substantially straight or forming a uniform curve at least over a section of the length of the shaft.

### DESCRIPTION OF THE DRAWINGS

In the following the invention will be described in further detail with reference to the drawing. The figures show ways of implementing the present invention and are not to be construed as being limiting to other possible embodiments falling within the scope of the attached claim set.
- Fig. 1 shows a perspective view of an electrode introducing electroporation device according to an embodiment of the invention.
- Fig. 2 shows, in a perspective view, a distal end of an embodiment of an introducer device according to the invention;
- Fig. 3 shows a section through a distal end of the introducer device shown in Fig. 2, the electrodes being in a retracted position;
- Fig. 4 shows a section through a distal end of the introducer device shown in Fig. 2, the electrodes being in an advanced position;
- Fig. 5 shows a perspective view of a distal end of the introducer device shown in Fig. 2, with an indication of the range of the advanced electrodes;
- Fig. 6 shows a partly cut-out sectional view of an electrode introducer device according to another embodiment of the invention
- Fig. 7 shows, in an exploded, sectional view, a distal end of an introducer shaft of a the introducer device shown in Fig. 6;
- Fig. 8, in an exploded view, shows details of the introducer device shown in Fig. 6;
- Fig. 9, in a frontal view, shows a distal tip of a device according to one embodiment of the invention, with two layers of extended electrode distal ends visible;
- Fig. 10 shows some of the electrodes extending from the distal tip of the device shown in Fig. 9, indicating a pulse emitting pattern between these electrodes; and
- Fig. 11 shows the resulting pattern of the electric field induced in a target tissue by the pulse emitting pattern Indicated in Fig. 10.

### EMBODIMENTS OF THE INVENTION

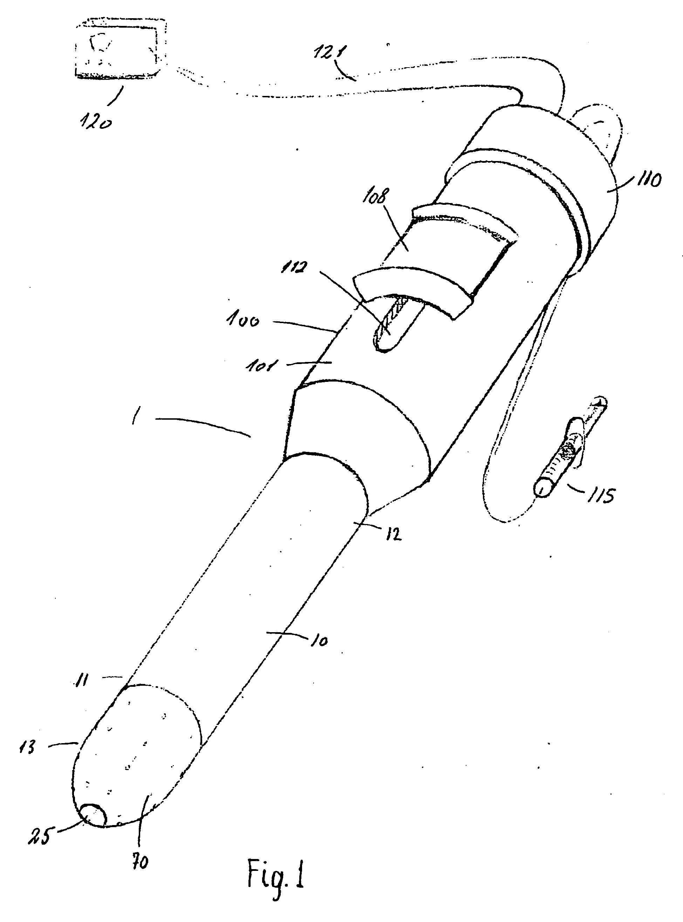

In fig. 1 an electrode introducing electroporation device 1 according to an embodiment of the Invention is shown. The device 1 comprises a handle section 100 and an elongate Introducer shaft 10 preferably having a length suitable for accessing deeper-lying tissue regions. The length of the shaft 10 may be adapted for the Intended use. The shaft 10 is attached to the handle section 100, and has a proximal end 12 adjacent to the handle section 100 and a distal end 11. The shaft may in one embodiment be fixedly attached to the handle section. In other embodiments the shaft may be detachably mounted to the handle section 100, and may comprise suitable means for establishing temporary connections, e.g. for conducting electrical pulses. A distal tip 13, that is preferably shaped to permit the creation of a channel through intervening layers of tissue while causing minimal damage to said tissue, is disposed at the distal end 11 of said shaft 10. The distal tip 13 has a rounded, non-cutting shape. In other embodiments (not shown) the distal tip may be provided with a cutting edge or a pointed tip, i.e. a sharpened tip. These latter embodiments are e.g. well-suited for percutaneous applications. In either case, the distal tip 13 may be formed integrally with the introducer shaft 10 or it may be a formed as a separate part coupled to the distal end 11 of the introducer shaft 10. With a removable/detachable tip 13, and/or a detachable shaft 10, the length and thereby the reach of the device, may be adapted, by a suitable choice of shaft. Further, this allows for use of single-use only parts for the parts that are inserted into a patient. Thereby, the need for disinfection of the parts to be inserted into a patient may be eliminated.

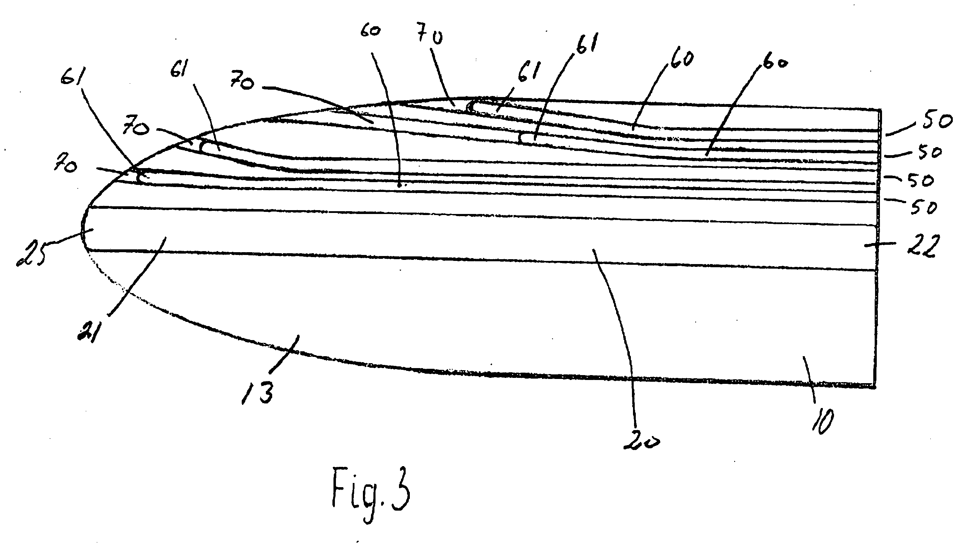

The introducer shaft 10 comprises a centrally located delivery channel 20 (see Fig. 3) provided through the shaft 10 from the proximal end 12 to the distal end 11 along a longitudinal axis, L, of said shaft 10, and terminating through said distal tip 13, said channel 20 having a proximal end 22 and a distal end 21. At the distal end 21 of the channel 20 one or more outlets 25 are provided in the distal tip 13 in order to administer an amount of fluid/medical compound adjacent to the distal tip 13. In the embodiments shown in the figures a single outlet 25 is provided, however, the channel 20 may split up into a multitude of minute channels at the distal end 21, each having an outlet at the distal tip 13. The proximal end 22 of the channel 20 extends through the shaft 10 to the handle section 100, and is adapted for connection to a drug/genetic material delivery means (115) comprising a storage of a drug/medicament and/or means (e.g. a pump or a piston or the like) for advancing said medicament from said storage and through said channel 20 to a target tissue. In a simple form the delivery means may be provided by a syringe 115, connected to the delivery channel 20 via the handle section 100, e.g. by a tubing.

In an alternative embodiment (not shown), the channel 20 may be configured to receive an elongate delivery system, e.g. in the form of a tubing, that may reach from the storage means into the region to be treated. Such a delivery system may comprise a syringe connected to said tubing, in such a way that the channel is adapted to receive e.g. a distal section of said tubing.

In yet another alternative embodiment (not shown), the device 1 may provide an integrated therapeutic molecule delivery system comprising delivery means with advancing/pumping means and/or a storage for a medicament/drug, isotope or a genetic material solution, being integrated in the handle section 100.

The electroporation device 1 and the delivery channel 20 may also be configured by e.g. appropriate coupling means and/or dimensioning to receive and guide for instance an ultrasound probe, a surgical tool or another tool for minimally invasive manipulation of tissue. Thus the device 1 can be used in a flexible way, where for example it is not necessary to remove the device 1 and replace it with another specialized surgical tool, if the operator/surgeon encounters unexpected obstacles/difficulties prior to, during or following the electroporation process.

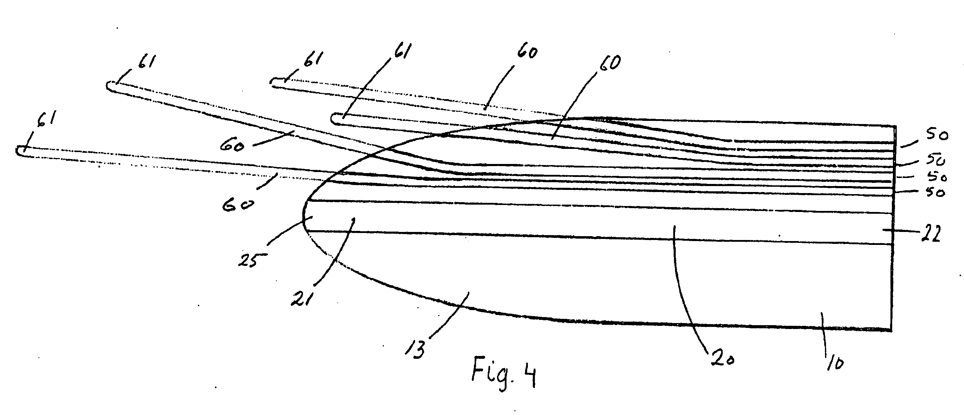

The shaft 10 further comprises a plurality of guide channels 50 (see Figs. 3 and 4), distributed around the central channel 20, and extending from the proximal end 12 to the distal end 11 of said shaft 10, and through the distal tip 13. Each guide channel 50 is adapted for guiding one or more elongate electrodes 60 that are movable relative to the shaft 10 between a first retracted position, as shown in Fig. 3, and a second extended position, as shown in Fig. 4.

In an alternative embodiment (not shown) each guide channel 50 may be provided, at least along a section of the shaft 10, by individual tubes, the shaft 10, in said section being formed by the set of individual tubes.

Each electrode 60 has a proximal end 62, extending into the handle section 100, a distal end 61 and an intermediate region 63 electrically connecting the proximal end 62 and the distal end 61 of each electrode 60.

The proximal ends 62 of the electrodes 60 are configured to act as connectors, thus providing a means of connecting the electrodes 60 to an electric stimulus generator 120 that supplies single electric pulses or sequences of electric pulses according to electroporation protocols for drug and gene delivery. The electric pulses are intended to generate an electric field for the purpose of creating transient permeabilization of cell membranes and/or an electrophoretic effect in the vicinity of the distal ends 61 of said electrodes 60 when the introducer device 1 is placed in or close to a target tissue area and the electrodes 60 are forwarded to an extended position, see further regarding the use of the device below.

The electrodes 60 are connectable to an external electric stimulus generator 120 via an electronic connector (cable) 121 at the handle section 120 as shown in Fig. 1. In an alternative embodiment an electric stimulus generator 120 may be formed integrated with the introducer device, preferably in the handle section 100.

The configuration of the proximal ends 62 of the electrodes 60 further permits movement of the electrodes 60 between a first retracted position and a second extended position in a deployment sequence that will be further described below.

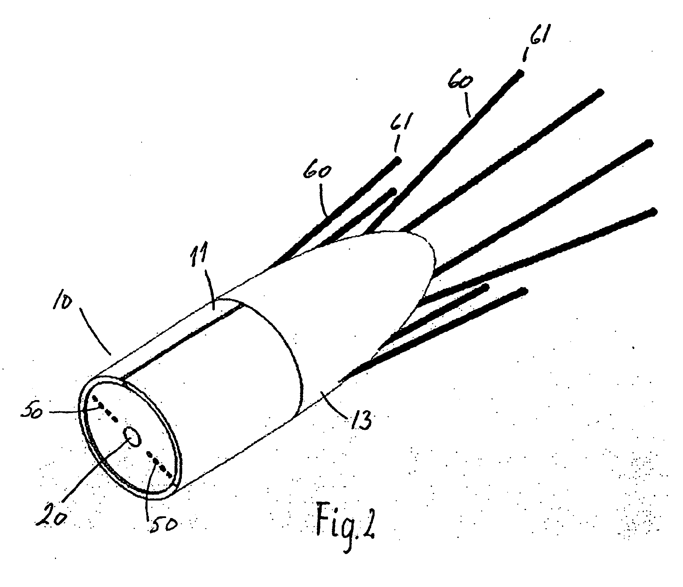

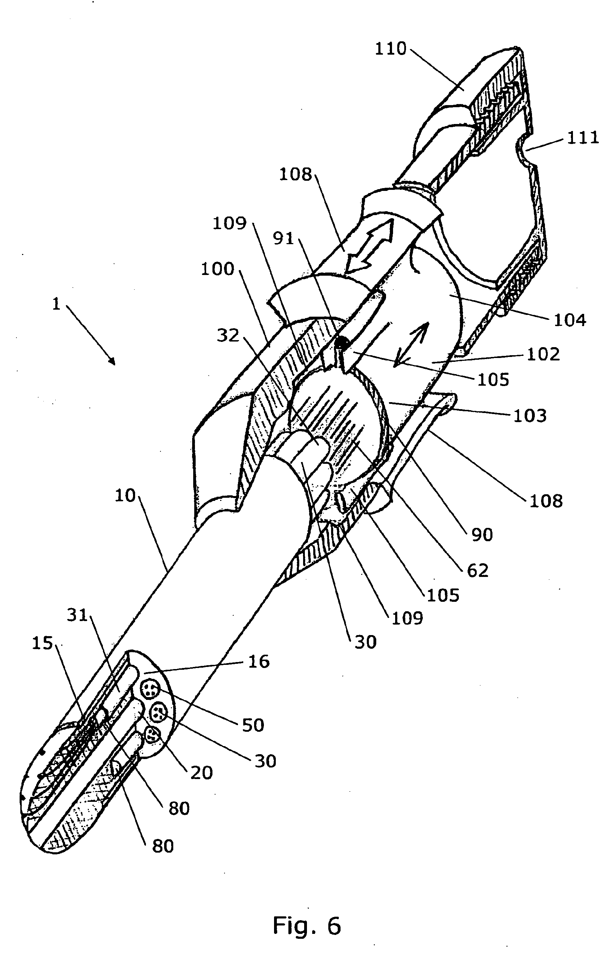

The intermediate regions 63 of the electrodes 60 are movably received in said electrode guide channels 50 running through the introducer shaft from the proximal end 12 to the distal end 11 at the distal tip 13. Preferably, each electrode 60 has its own channel 50 to support and protect it and Insulate it from the other electrodes 60, as shown In Figs 2-4, but multiple channels 50 may be bundled together In electrode assemblies, for example as shown in Fig. 6. Said electrode guide channels 50 permit longitudinal movement of the electrodes 60 between the first retracted position and the second extended position.

Electrode end points at the distal ends 61 of the electrodes 60 are movably received in distributor channels 70 formed in the distal tip 13, and extending to the outer surface of said distal tip 13. Each distributor channel 70 further communicates with a corresponding guide channel 50 in the shaft proper 10. Thus, movement of the electrodes 60 in a longitudinal direction (with respect to the longitudinal axis of the shaft 10) between a first retracted position where the distally disposed end points 61 of the electrodes 60 are concealed within the distal tip 13, and a second extended position, where the end points 63 of the electrodes 60 are extended from the distal tip 13, is allowed.

In an alternative embodiment (not shown), the device may only have distributor channels 70 formed in the distal tip 13, the electrodes 60 being contained in a hollow shaft 10, the Individual channels 50 being left out.

While positioned in the first retracted position, which is the default mode of the device 1, the end points at the distal ends 61 of the electrodes 60 are held in storage in the distributor channels 70 in the distal tip 13, thus permitting the minimally invasive Insertion of the device 1, i.e. with minimal damage to surrounding tissue.

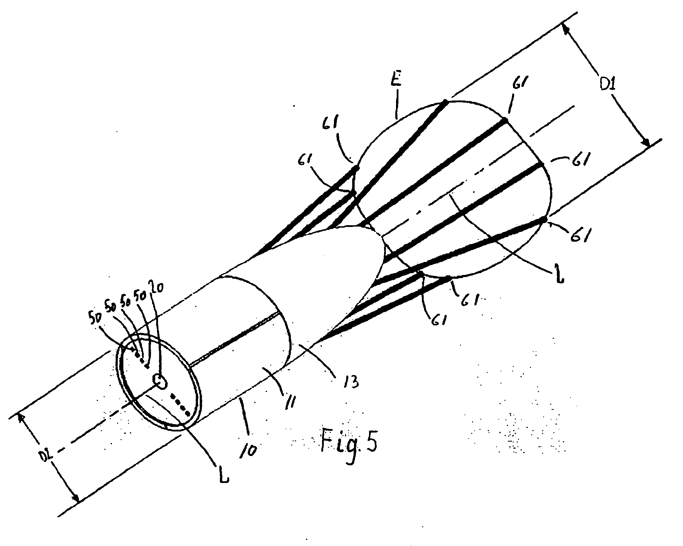

The distributor channels 70 are shaped to ensure deployment of the distal ends 61 of the electrodes 60 in a predetermined pattern where a largest distance D1 (See Fig. 5) between a pair of oppositely arranged electrode end points 61, in a plane transversal to the longitudinal axis of the introducer shaft is larger than the diameter - or the largest extension D2 of the introducer shaft 10/distal tip 13 - in a plane perpendicularly to the longitudinal axis of the introducer shaft 10. Thus, it is made possible to access deeper lying tissues, e.g. within the brain, through a single channel using a single Introducer shaft 10, spreading the intervening tissues during the Insertion, and, when the tip 13 reaches the target tissue, the electrodes can be extended through and/or around the target tissue. This allows an operator (surgeon) to treat a target tissue region or volume which has a cross-sectional dimension/extent larger than the diameter of a cross-section of the introducer shaft 10, where the cross-section is taken in a plane perpendicular to the longitudinal axis of the introducer shaft 10. In order to provide the above described distribution of the distal ends 61 of the electrodes 60, the distributor channels 70 are formed such that at least some of the distributor channels 70 curve outwardly, i.e. away from a longitudinal centre axis L of the introducer shaft 10 (as seen from their connection to the distal end 11 of the corresponding guide channels 50 in the shaft 10 and towards the outer surface of the distal tip 13 where the distributor channels 70 terminates). Each of the distributor channels 70, or sets of distributor channels 70may be provided with a different individual shape/deflection/curving in order to ensure a specific pattern or distribution of the extended electrodes 60 during use.

Alternatively, the deflection away from said longitudinal axis L may be provided by e.g. a pre-tensioning or biasing of said electrodes 60. Such tensioning may be provided by a suitable choice of materials, e.g. a shape memory alloy such as Nitinol, or by forming the (flexible) electrode e.g. in a bent shape, such that when it is arranged in a straight guide channel 50 of the shaft 10 it is held in tension. The individual electrodes 60 or set of electrodes may have an individual biasing such that the electrodes may, when extended from their retracted position in the shaft 10/tip 13 form a desired spatial pattern around the target tissue.

Further, the desired spatial distribution of the part of the electrodes extending from the tip 13 may be provided by a combination of the shape of the tip distributor channels 70 and a biasing of the electrodes 60.

In use, the electronic connection means (not shown) at the proximal ends 62 of the electrodes 60 are connected to a suitable electric stimulus generator 120. The shaft 10 of the introducer device 1 is then inserted, e.g. through a bore hole in a patient's skull or an incision in the patient's skin and introduced to the target region of the patient's body. The precise location of the target region and thereby for the bore/incision may be identified by means of ultrasound, CT, MR or another suitable means, and the correct position of the tip 13 of the introducer shaft 10 (post insertion) may be verified by similar means prior to, during or after deployment of the electrodes. When a correct position of the tip 13 of the introducer shaft 10 has been obtained relative to the target tissue, an operator may deliver a suitable chemotherapeutic agent, in fluid or liquid form, or a dose of genetic material or other substance through the delivery channel 20 and into the tissue region to be treated.

Before, during or after delivery of the drug or genetic material through the delivery channel, the operator may deploy some or all the elongate electrodes 60 in a desired pattern. Deployment is performed by actuating a suitable deployment mechanism at the handle section 100 or at the proximal end 12 of the shaft 10, and results in the longitudinal motion of all or some the electrodes 60 along the axis of the introducer shaft 10 from the first retracted position - as shown in Fig. 3 - to the second advanced position, e.g. as shown in Fig. 4. The distributor channels 70 in the distal tip 13 may be shaped to provide each individual electrode 60 with a unique path through the tissue, when advanced from the tip 13, which enables the creation of an electrode pattern where a distance D1 between oppositely arranged electrode end points 61 in a plane transversal to the longitudinal axis of the introducer shaft 10 is larger than a diameter D2 (or the largest extent of the shaft 10 in a section perpendicular to the longitudinal axis of the shaft 10 if the shaft is not of circular cross section) of the introducer shaft 10 in the same transversal plane.

Upon deployment of some or all of the electrodes to their extended position, an operator may actuate the electric stimulus generator 120 to deliver one or more pulses, e.g. a sequence of short and intense pulses to the tissue to be treated (target tissue). To ensure a suitable distribution of pulses and the thereby induced electric fields in the target tissue, pulses may be assigned to alternating specific electrodes 60 in a sequential pattern that may be tailored to suit the anatomy of the individual region of the body to be treated and/or the geometry of the specific malignant target tissue. Such assignment may be obtained for instance by suitable manipulation of the electric stimulus generator, e.g. through programmable electronic control means.

Upon pulse delivery, the operator may retract the elongate electrodes 60 to their retracted position by suitably manipulating the deployment mechanism in the handle section 100, and the device may be removed from the body of the patient. Alternatively, the operator may reposition the device 1 after having retracted the elongate electrodes 60, potentially permitting multiple pulse applications covering a larger area in a single device insertion.

The electrode introducer device 1 shown in Figs. 2-5 is depicted as having eight guide channels 50, distributor channels 70 and electrodes 60. However, a device according to the invention may be provided with any number of electrodes 60. The distribution of the guide channels 50 over a cross-section of the introducer shaft 10 shown in Figs. 2-5, is such that the electrodes all run in a plane parallel to the longitudinal axis of the introducer shaft 10. However, the electrodes 60 and their guide (and distributor) channels 50 (70) may be located around the entire circumference of the channel 20 in the shaft 10, surrounding the delivery channel 20 in other patterns as well.

Each electrode is formed in an electrically conductive material. Parts of the electrodes may be formed with an electrically insulating coating or sheathing, such that only the most distal ends 61 (points) of the electrodes 60 are un-insulated. Thus, the electric pulses will create an electric field spanning the distance from point to point (distal end 61 to distal end 61), and a readily controllable firing pattern and thus a more controllable and accurate electric field may thus be generated by suitable selection and assignment of electrodes. For completeness it is to be understood that the entire length or part of the entire length of the electrodes 60 may also be electrically un-insulated, provided that the guide channels 50 and the distributor channels 70 are formed in an electrically insulating material.

As shown in Fig. 5 the device may be configured such that the distal ends 61 of the electrodes 60 may form an ellipsoid field E that is the result of this electrode 60 pattern. A target tissue could be imagined situated within the ellipsoid area E, shown in the figure. Some of the electrodes 60 are thus advanced through the target tissue when guided to their extended position. In other embodiments of the invention it can be imagined that electrode patterns can be formed, such that a target area can be surrounded by electrode points (distal ends) 61 in various three-dimensional patterns, e.g. a spherical or spherically elliptic or ellipsoid pattern.

As can be appreciated from Fig. 5, a device according to the invention may be adapted with sets of electrodes 60 that may be extendable to different distances from the distal tip 13 along the longitudinal axis of the shaft 10, such that the distal ends 61 of each set are positioned in a common plane perpendicular to the longitudinal axis of the shaft 10. In Fig. 5 four sets of two electrodes extend to different distances from the distal tip 13, thus forming the above mentioned ellipsoid shape E.

Further, in a non-claimed embodiment some of the electrodes 60 may be formed in such a way that they undertake a curved path through the tissue such that when advanced forward towards their extended position they will initially be deflected away from the central longitudinal axis L of the shaft 10, and will then reflect back such that the distal tip closes in on the central, longitudinal axis of the shaft 10, when advanced further. Thus, when fully extended, such an electrode 60 will describe a gently U-shaped or substantially, softened Ω-shaped curve. This may be accomplished by providing electrodes in an elastic material or a shape memory alloy such as Nitinol or by providing different section (lengthwise) of the electrodes with different biases (pre-tensionings).

Yet further, guiding channels may be shaped to impose on the electrodes certain paths through the tissue. A strictly linear path through the tissue is imposed on the electrodes, as the electrodes will then be able to withstand much higher loads without buckling - as opposed to electrodes given a curving path.

The deployment mechanism for the electrodes 60 may be manually driven or motorized (e.g. electronically controlled). The deployment mechanism may be adapted to advance all electrodes simultaneously as a set, or individually, or in groups (subsets) of electrodes 60. When the electrodes are advanced simultaneously, different electrode patterns may be achieved through a predetermined composition of electrodes of suitable lengths, shapes (by tensioning, alternative cross-sections predisposing the wire for certain directions of movement or by adequate shaping of guide channels) and materials. The device 1 according to the invention may further be controlled by an electronic control unit (not shown), either incorporated in the device 1 or connectable to the device 1 through a cable or a wireless connection. In the wireless configuration, a suitable power supply is preferably located inside the device. The electronic control unit may be programmable, such that a desired electrode pattern may be programmed prior to a surgical procedure.

In alternative embodiments (not shown), and as mentioned above, a partially disposable device variation of the above described embodiments is proposed, with a disposable introducer shaft 10 and non-disposable (re-usable) handle section 100 comprising a deployment mechanism with interfaces to electrodes formed in the disposable introducer shaft 10 and a electronic connections that may be customized to individual electrical stimulus generators 120.

The shaft may in all embodiments be formed in a plastic or metallic material such as titanium, stainless steel or an injection moulded polymeric material. The outer diameter of the shaft is preferably five (5) millimetres or smaller, preferably between gauge 17 to 14 incl. The wall thickness of the shaft is preferably between 0.05 mm and 0.25 mm. The guide channels 50, 70 may be formed in a suitable material, e.g. formed in a thermoplastic elastomer or a similar electrically insulating material. The electrodes 60 may be formed in an electrically conductive material such as titanium, stainless steel or the like

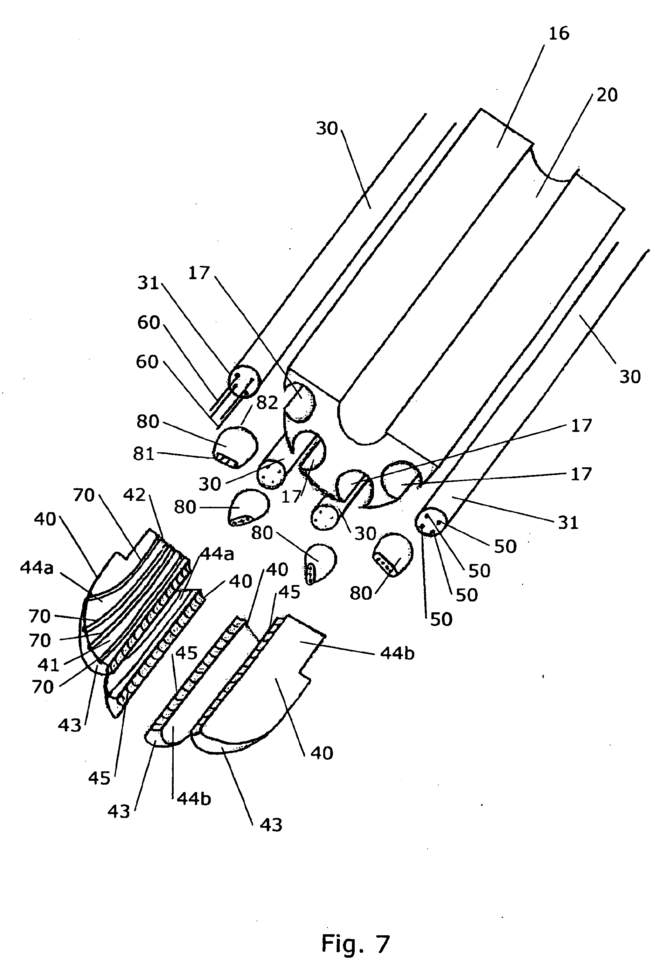

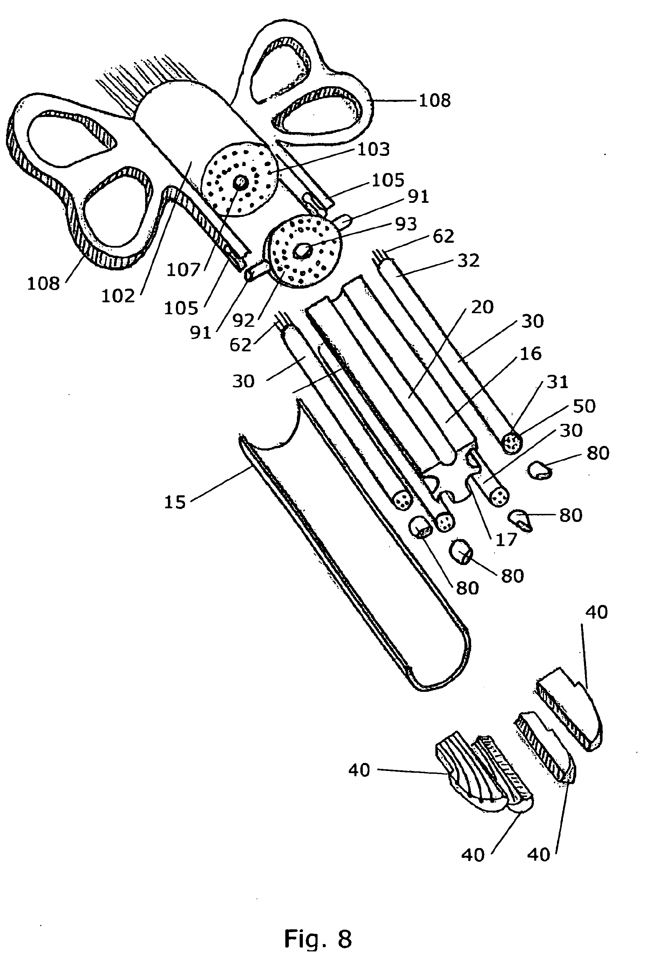

In the following, an aspect of the invention, suited in particular for applications within the brain, e.g. in the treatment of brain cancer or genetic deficiencies will be described in further detail with reference to Figs. 6-8. Like references will be used for similar parts, with respect to the aspects of the invention shown in the previous drawings. The electrode introducer device 1 comprises an introducer shaft 10 and a handle section 100. The introducer shaft 10 is intended for insertion into the body of the patient and is fixedly attached to the handle section 100.

In alternative embodiments a partially disposable device is proposed, with a disposable introducer shaft 10 and non-disposable (re-usable) handle section 100 comprising a deployment mechanism with interfaces to electrodes formed in the disposable introducer shaft 10 and a connector that may be customized to individual electric stimulus generators 120.

The introducer shaft 10 comprises the following:
- An outer tube 15 having a proximal end 11 and a distal end 12 which is preferably formed in a plastic or metallic material such as titanium, stainless steel or an injection moulded polymeric material. The outer diameter D2 of this tube is preferably five (5) millimetres or smaller. The wall thickness of said outer tube is preferably between 0.05 mm and 0.25 mm and the length of the tube is preferably between 50 mm and 500 mm depending on the particular application.
- An inner electrode assembly guide 16 that is preferably formed in a thermoplastic elastomer or a similar electrically insulating material. The inner electrode assembly guide 16 is placed in an inner lumen of the outer tube 15. The electrode assembly guide 16 has a flattened proximal end and a flattened distal end comprising faces that lie perpendicular to the longitudinal axis. This electrode assembly guide 16 comprises eight straight, semi-open channels 17 distributed in a circular pattern around and partially sunk into an outer periphery of the electrode assembly guide 16 and running in parallel tracks from the proximal end 12 to shortly before the distal end 11. In addition, the electrode assembly guide 16 has a central bore/delivery channel 20 providing a fluid channel and/or a working channel for surgical instruments. The outer periphery of the electrode assembly guide 16 fits within the lumen of the outer tube.
- Eight electrode assemblies each comprising a cylindrical guide sheath 30. The guide sheaths 30 are preferably formed in a thermoplastic elastomer or a similar electrically insulating material, and are received in the straight semi-open channels 17 in the electrode assembly guide 16 and firmly attached therein. The cylindrical guide sheaths 30 have a flattened proximal 32 and distal end 31. The interior of each electrode assembly guide sheaths 30 comprises four mutually electrically insulated electrode channels 50 running in parallel from the proximal 32 to the distal 31 end, and distributed in a pattern that resembles a square with the electrode channels 50 placed in the corners. The proximal end of each electrode channel 50 comprises an electrode support zone with a slightly Increased diameter for the first approximately 20 mm, to receive a corresponding supporting sheath that is mounted on the proximal end 62 of each electrode 60. Further, the electrode assemblies comprise a total of thirty-two elongate, preferably cylindrical electrodes 60 formed in an electrically conductive material such as titanium, stainless steel or the like, each electrode having proximal ends 62, distal ends 61 and Intermediate zones 63. Approximately 20 mm from the proximal 62 end of each electrode 60, a supporting sheath (not shown) 20 mm long may be provided, the sheat surrounding a part of the intermediate zone 6 of the electrode 60. This supporting sheath is meant to lend support to the individual electrodes to prevent buckling or bending during the deployment sequence and is configured to slide into the electrode support zone (of the electrode channels 50 on the guide sheaths 30) when the electrode is moved from its retracted to its advanced position during deployment. Each electrode 60 is preferably covered with an electrically insulating layer except on the distal tip which is left without insulation. Yet further, the electrodes 60 are grouped in groups of four, and each group of electrodes is inserted in a cylindrical guide sheath 30, one electrode in each electrode channel 50. Insertion is done so that the proximal ends 62 of the electrodes 60 protrude approximately 30 mm from the proximal ends of the guide sheaths 30, whereas the distal ends 61 of the electrodes 60 protrude approximately 40 mm from the distal ends of the guide sheaths 30.
- Eight alignment bushings 80, each configured to receive and guide four electrodes 60 and each with a proximal end 82 and a distal end 81 and four alignment channels 83. The alignment bushings 80 are placed in extension of each of the eight electrode assemblies (guide sheaths 30), and are configured to interface with said assemblies and guide sheaths 30 and to receive the four elongate electrodes 60 where they emerge from the distal ends 31 of said assemblies/guide sheaths 30 in a manner to prevent electrode buckling or bending during the deployment sequence. To achieve this, the proximal end 82 of each alignment bushing 80 is configured to align the four alignment channels 83 with the four electrode channels 50 of the electrode assemblies/guide sheaths 30. The path of the alignment channels 83 of each alignment bushing 80 is configured to change the pattern of the elongate electrodes from the square pattern configuration when emerging from the electrode assemblies/guide sheaths 30 to a linear pattern when they emerge from the alignment bushing 80. Since the eight electrode assemblies/guide sheaths 30 are distributed in a circular pattern and the eight alignment bushings 80 are placed in extension of the assemblies, a radial pattern may be created by suitably orienting the alignment bushings 80.
- A distal tip 13 that is an immediate extension of, and aligned with, the electrode assembly guide 16. The distal tip 13 comprises eight elongate, roughly triangular spacer units 40, each with a proximal end 42 and a tapered, rounded distal end 41, a rounded outer surface 43 and an inner section with two faces 44a, 44b. One face 44b is smooth and one face 44a comprises four distributor grooves 70 that run from the proximal end 42 towards the distal end 41 while curving towards the outer rounded surface 43 of the spacer unit 40, each in a predetermined unique curve. The faces 44a, 44b meet in a 45 degree angle to create a wedge. A rounded cut-out 45 takes away the sharpened end of the wedge. The proximal ends 42 of the spacer units 40 have a reduced height and are inserted into the distal end 11 of - and held tightly together by - the outer tube 15 while the distal ends 41 of the spacer units 40 meet to form a torpedo-shaped tip 13. When all eight wedge-shaped spacer units 40 are held together by the outer tube 15, the rounded cut-outs 45 create a central bore 46 aligned with the delivery channel 20 of the electrode assembly guide 16. The spacer units 40 are oriented so that the smooth face 44b of one spacer unit 40 rests against the face 44a comprising four distributor grooves 70 of the neighbouring spacer unit 40, thus creating four distributor channels 70 per spacer unit 40, for a total of 32 channels. Each distributor channel 70 is configured to receive a specific elongate electrode 60 where it emerges from its respective alignment bushing 80 and to permit its longitudinal movement between a first retracted and a second advanced position (in the same manner as shown in Figs. 3 and 4 respectively). In their first retracted positions, all electrodes 60 are placed with their distal ends 61 entirely within the distributor channels 70. When the electrodes are advanced as part of a deployment sequence, the distal ends 61 of the electrodes 60 are moved out of the distributor channels 70 to protrude from the distal tip 13. As the grooves and thus the channels 70 lead towards the rounded outer surface 43 of each spacer unit 40 (and thus are deflected away from the longitudinal axis of the introducer shaft 10) and each in its own angle, each electrode is given its own path and emerges from the distal tip 13 in its own direction when advanced. Thus, by providing 32 electrodes that may be moved between a first retracted and a second advanced position, each with a unique path that leads away from the distal tip 13 and ends in a unique point it is possible to generate a three-dimensional pattern of electrode points 60 as previously described.
- A round adaptor plate 90, fixedly attached to the proximal ends 62 of the elongate electrodes 60 and placed proximally to the proximal end of the electrode assembly guide 16. The adaptor plate 90 is longitudinally movable between a first retracted and a second advanced position. The proximal ends 62 of the elongate electrodes are inserted in holes 92 in the adaptor plate 90 that are placed in a pattern resembling that of the electrodes 60 when they emerge from the guide sheaths 30 and the supporting sheath of each electrode is fixedly attached to the adaptor plate 90. The adaptor plate 90 further comprises a central hole 93 that is aligned with the delivery channel 20 of the electrode assembly guide 16, as well as two guide pins 91 that are placed oppositely to each other on - and protruding from - the outer periphery of the adaptor plate 90.

The handle section 100 comprises the following:
- A generally cylindrical housing 101 that is preferably formed in plastic or another suitable material. The housing comprises two half sections, each having an inner and an outer surface, a proximal end, a distal end and an intermediate zone.
- A deployment slider 102 that is preferably made of plastic or a similar nonconductive material and is movable between a first retracted and a second advanced position within and relative to said housing 101. The deployment slider 102 has a proximal end 104 and a distal end 104 and is in operative connection with the adaptor plate by means of two connecting clamps 105. Said connecting clamps 105 are configured to engage the guide pins 91 of the adaptor plate 90 and are slidably held in grooves 109 in the housing 101. The distal end of the deployment slider comprises 32 connections 106 that are configured to receive the proximal ends 62 of the electrodes 60 as they emerge from the adaptor plate 90. Said connections 106 are electrically connected to the distal ends of flexible leads (not shown) that conduct electric pulses from the electric stimulus generator 120 to the electrodes 60. The proximal ends of said leads are connected to a connector plug that constitutes an interface to an electric stimulus generator 120. The deployment slider 102 further comprises a central bore 107 aligned with the central hole 93 in the adaptor plate 90, as well as two or more finger grips 108 that protrude radially away from the outer surface of the housing 101, through openings in the same. Said finger grips 108 permit an operator to move the deployment slider 102 between a first retracted position and a second advanced position, in order to advance the electrodes 60. The distal half ends of the housing 101 are fixedly attached to the introducer shaft 10 so that the proximal part of the shaft 10, as well as the adaptor plate 90 and the deployment slider 102, all lie within the housing 101. Towards the distal part of the inner surface of each half section of the housing 101 is a groove 109 that is configured to receive one of two connecting clamps 105 of the deployment slider 102. In a proximal continuation of said groove 109 is placed a motion control slot 112 (see Fig. 1) that runs to the proximal end of each half section. The motion control slot 112 is configured to receive one of two finger grips 108 of the deployment slider 102 and permit longitudinal motion of the slider 102 between a first retracted and a second advanced position. The proximal end of the housing 101 is threaded to receive an end cap 110 that serves the dual purpose of closing the handle section 101 and holding the proximal ends of the two half sections of the housing 101 together. Further, one half section comprises an outlet configured to receive the leads 121, 122 as they emerge from the deployment slider 102.
- An end cap 110 that comprises an outer shell with a threading on its inner surface and an inner support cylinder that has a circumference corresponding with the circumference of the inner surface of the housing. The end cap further comprises a central hole 111 that is aligned with the central bore in the deployment slider 102 and is configured to receive the tubing of the drug dispenser.

In use, the connector plug of the device is connected to a suitable electric stimulus generator 120. The device 1 is then inserted through a bore hole in the patient's skull and introduced to the target region of the patient's body/brain. The precise location may be identified by means of ultrasound, CT, MR or another suitable means, and the correct position of the introducer shaft 10 prior to deployment may be verified by similar means. As described above, in other embodiments, the stimulus generator may be integrated in the handle section.

When a correct position of the introducer shaft 10 has been obtained, an operator may deliver a suitable chemotherapeutic agent or dose of genetic material through the central channel 111, 107, 93, 20 and into the tissue region to be treated. Delivery is done by inserting the elongate, length-adjusted and properly dulled needle of a syringe 115 in the central hole of the end cap and advancing it until no further motion is possible. The operator may then empty the syringe barrel 115 by pressing the syringe plunger, whereupon the liquid in the syringe is expelled into the tissue to be treated.

Before, during or upon delivery, the operator may deploy the elongate electrodes 62 in a predefined pattern. Deployment is done by moving the deployment slider 102 from its first retracted position towards its second advanced position until further movement is prevented by the end of the motion control slots 112. Said movement results in the motion of the electrodes 60 from the first retracted to the second advanced position. The distributor channels 70 in the distal tip 13 are shaped to provide each individual electrode 60 with a unique, preferably essentially linear path through the tissue and a unique end-point, and the goal is to enable the creation of an electrode pattern that may have a larger diameter (or maximum extent in a plane perpendicular to the longitudinal axis of the shaft 10) than the introducer shaft 10 and may ensure optimal distribution of the short and intense pulses and the thereby derived electric fields in the tissue to be treated. In one particular preferred embodiment the un-insulated electrode tips (distal ends 61) are positional and positioned with their end-points at least partially surrounding or enclosing the target region of tissue in such a way that the distal ends 61 describe or define the outer periphery of a spherical/spatial ellipse. In said preferred embodiment the 32 electrodes are organized in four layers, each layer having a different diameter and consisting of eight electrodes 60 with their end-points (distal ends 61) describing a circular pattern in a plane perpendicular to the axis of the introducer shaft 10.

Upon deployment, an operator may activate the electric stimulus generator 120 to deliver a sequence of preferably short and intense electric pulses, for example square-wave pulses, to the tissue to be treated. To ensure a suitable distribution of pulses and the consequent electric fields in the tissue to be treated (target tissue), pulses may be assigned to alternating specific electrodes 60 in a pattern that may be tailored to suit the anatomy of the individual region of the body to be treated and/or the geometry of the specific malignant target tissue. In an embodiment, at least some of the end-points 61 of the electrodes 60 are placed in equidistant relation to other electrode end points 61, and at least some pulses are assigned to equidistant pairs of electrodes. Thus, a homogenous or heterogeneous, controllable three-dimensional electric field can be created in the target tissue.

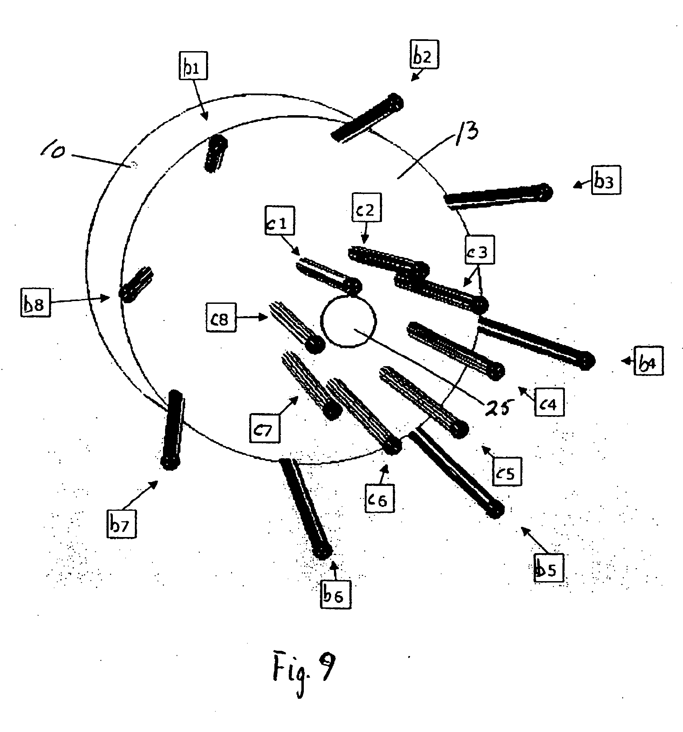

In a further embodiment the un-insulated electrode 60 tips are positionable in such a pattern that their end-points 61 outline an outer periphery of an ellipsoid or an ellipse in a plane taken parallel to the longitudinal axis of the shaft 10 - corresponding to what is illustrated by reference E in Fig.5. In this embodiment, and as further shown in Fig. 9, the 32 electrodes 60 are organized in four substantially parallel layers (in a plane perpendicular to the longitudinal axis of the shaft 10) numbered a-d, (a being the top-most (with respect to the distal tip 13)/most-distal layer (with respect to the user/surgeon)) consisting of eight electrodes numbering 1-8 in each layer, with their end-points describing an elliptical or a circular pattern perpendicular to the axis of the introducer shaft. In Fig. 9, the top layer a and bottom layer d of electrodes 60 has been left out, for the purpose of clarity, such that the b (b1-b8) and c (c1-c8) layers are shown.

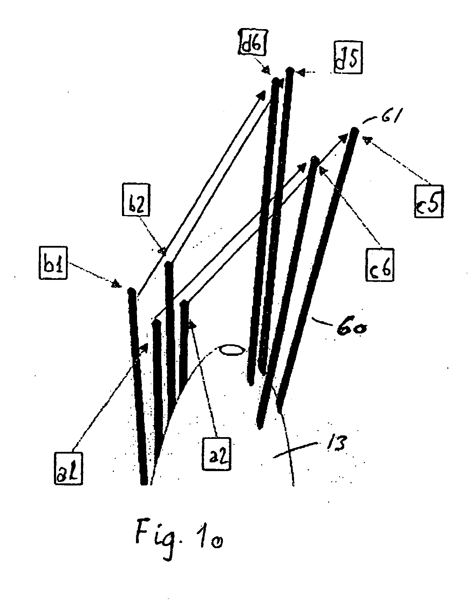

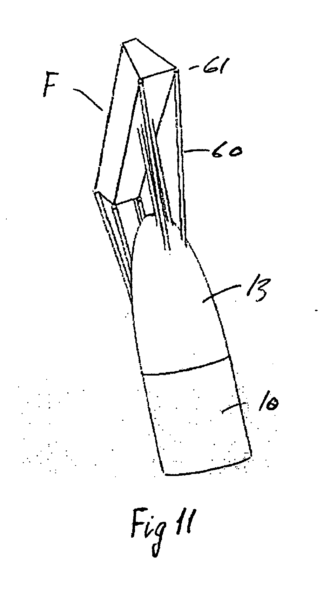

The efficiency of the electroporation may be enhanced by adapting a controlled pulse emitting sequence, thus creating a controlled electric field. In one suggested pulse sequence, at least some of the pulses assigned travel from electrodes in layer a to electrodes in layer c that are placed in equidistant relation to the electrodes in layer a, while others simultaneously travel between equidistant pairs in layer b and layer d. In one particular firing sequence, pulses travel from positive electrodes a1 and a2 to negative electrodes c6 and c5, and simultaneous pulses travel from positive electrodes b1 and b2 to negative electrodes d6 and d5, as illustrated in Fig. 10 where only the mentioned electrode distal ends 61 are shown, the other 24 being removed for the sake of clarity. The pulses will travel the shortest possible way (assuming uniform electric resistance in the target tissue) wherefore the electric field can be shaped and controlled by the positioning of the electrodes such that firing between the electrodes in different layers can be made between equidistant positive and negative pairs of electrode ends (61) (point electrodes). Thus, an elongate, three-dimensional electric field F is generated, as shown in Fig. 11. The position of the field may be altered to cover the largest possible tissue volume by sequentially changing the assignment of pulses to other equidistant positive and negative electrodes in a suitable pattern.

Upon pulse delivery, the operator may retract the elongate electrodes 60 to their first retracted position by moving the deployment slider 102 from the second advanced position to the first retracted position whereby the electrodes are retracted to their default position within the distal tip 13, and the device 1 may be removed from the body of the patient. Alternatively, the operator may reposition the device after having retracted the elongate electrodes 60, potentially permitting multiple pulse applications covering a larger area in a single device insertion.

In either of the above embodiments a separate channel (not shown) or a portion of the delivery channel 20 may be used to deliver a saline solution to enhance the Electroporation process by increasing tissue conductivity. A saline solution may also be introduced via the delivery channel 20 proper. In either case suitable means for connecting the channel 20 to a source of saline solution may preferably be provided at the handle section.100

As described above, the cross-sectional shape of the electrodes is preferably essentially circular. However, in other embodiments, other cross sectional shapes may be applied. The diameter and cross-sectional shape of the distributor channels 70 are in any event preferably dimensioned for the desired electrode diameter and cross-sectional shape, in order to provide the best possible support for the electrodes, without limiting their ability to be moved from their retracted position to their extended position (and back).

In either of the above described embodiments, the electrode diameter is preferably 0.4 mm or smaller, such as 0.3 mm, 0.25 mm including electrically insulating coating. The diameter of the electrodes 60 is typically correlated to the stiffness of the electrodes, such that the thicker the electrode, the stiffer the electrode. For some applications a stiff electrode may be necessary, e.g. if the tissue is tough. In soft tissue a less stiff electrode may be applied.

Also depending on the application, the tip of the electrodes may be configured such that it may cut through tissue or it may be smooth in order to more gently spread the tissue.

Further, the electrodes may biased (e.g. pre-tensioned) in such a way that their geometrical configuration in their extended state varies with the extent to which they have been extended beyond the distal tip 13 of the shaft 10. This may be applied be providing the electrodes 60 with different tension characteristics along the lengthwise direction of the electrodes. Thus, a very flexible electroporation device may be obtained.

In the description above and in the drawings, the delivery channel 20 has been illustrated to be centrally located within the shaft 10. However the delivery channel 20 may be asymmetrically located within the shaft, with respect to its cross sectional position. In other embodiments (not shown) the single delivery channel 20 may be replaced by a plurality of smaller delivery channels, each having an outlet at the tip 13. Thereby a more even distribution of an injected therapeutic molecule solution can be obtained.

As described above, a surgical tool or the like may be inserted via the delivery channel 20. The invention also concerns a combination of an electroporation device having a delivery channel according to any of the embodiments described above and an therapeutic molecule solution injection device. The therapeutic molecule solution injection device comprises an elongate hollow part adapted for the delivery channel 20, and a steerable outlet tip. The elongated hollow part is adapted in length, such that the steerable outlet tip can be extended beyond the tip 13 of the electroporation device. The steerable outlet tip may be used to administer a dose of therapeutic molecule solution in a precise location in the target tissue.

Alternatively, or in addition to the combination with therapeutic molecule solution injection device, the electroporation device may have a steerable tip 13. This may be provided by having control rods or strings extending through the shaft 10 to the tip 13, the tip e.g. being pivotally mounted at the distal end of the shaft 10, pivotably about an axis either parallel to the elongate axis of the shaft or perpendicular (or at another angle) to the axis of the shaft. The extent to which the tip 13 may be steered is of course dependant on the stiffness of the electrodes, and a flexible alignment between the channels 50 in the shaft and the channels 70 in the tip 13. By providing a steerable tip 13, the flexibility and reach of the electroporation device may be enhanced, since for also a larger target tissue volume, a single entry hole/channel, formed by the shaft 10 through the surrounding (healthy) tissue is necessary. Thus the reach of the electrodes may be expanded by a turning of the tip 13 or a combination of a turning of the shaft and a tipping of the tip 13 (when the electrodes are in retracted position in the shaft) Thereby the applied electrical field can be repositioned, in a sequence until the entire target tissue may be covered. Further the direction of the outlet of the delivery channel may be altered in order to provide for a more precise delivery of a therapeutical molecule solution. The steerable tip 13 may be combined with the above mentioned therapeutic molecule solution injection device in order to further enhance the reach and flexibility of the drug delivery. However, the steerable tip 13 may also be applied in embodiments without a delivery channel, i.e. embodiments suitable for systemic introduction of drugs or for irreversible electroporation.

The electrodes may also be prepared with/covered by/impregnated with a drug or DNA molecule compound that may be dissolvable in an electrical field. Thereby, a drug etc. may be released from the electrodes when an electrical field is applied to the target tissue via the electrodes. Thereby the delivery channel 20 may be spared. However, the drug impregnated electrodes may also be used with embodiments having a delivery channel 20 in order to release multiple drugs or in order to save the delivery channel for e.g. a field enhancing saline solution as described above.

The Electroporation device according to the invention and as described above may in various embodiments be applied for an electroporation method comprising the steps of
- providing an electroporation device 1 comprising
   - an elongate introducer shaft 10 having a distal tip 13 formed with a substantially smooth, rounded, non-cutting shape, with a substantially smooth, nun cutting transition to the introducer shaft 10; and
   - a set of electrodes 60 having respective distal ends 61, each electrode 60 being slidably arranged within said introducer shaft 10 from a retracted position, where said distal ends 61 are enclosed within said introducer shaft 10, to an extended position, where said distal ends 61 extend from said distal tip 13,
- inserting said introducer shaft 10 through tissues of a body and bring said distal tip 13 into a vicinity of a target region to be treated, while said electrodes 60 are in said retracted position;
- extending said electrodes 60 to an extended position such that the electrode tips 61 are in a position distally of said distal tip 13, and such that said electrode distal ends 61 are deflected away from a longitudinal axis L of said shaft 10 in such a way that at least one planar projection taken in a plane perpendicular to said longitudinal axis L of a distance between a pair of distal ends 61 of said electrodes 60 is larger than a maximal extent of a cross-section of said introducer shaft 10, said cross-section taken in a plane perpendicular to said a longitudinal axis L at a distal end 11 of said introducer shaft 10, the deflection of said distal ends 61 of said electrodes 60, when in their extended position, being provided by a curving of distributor channels 70 formed in said distal tip 13; and
- applying through said electrodes 60 one or more electric pulses to the target region tissue to create a transient permeabilization of cell membranes of tissue in said target region.

In a variation of this method each of said electrodes 60 may be extended such that their distal ends 61 form a spatial distribution at least partly around a volume of target tissue.

In a variation of this method said electrodes 60 may be extended individually or in sets to their extended positions to a spatial configuration of the distal 61 at least partially surrounding a target tissue.

In a variation of this method said electrodes 60 may be extended such that their distal ends 61 form a substantially spherical distribution pattern.

In a variation of this method a subset of said electrodes 60 may be extendable, such that their distal ends 61 form an ellipsoid pattern E in a plane parallel to a longitudinal axis L of the shaft 10 when extended.

In a variation of this method, the method comprises a step of administering a dose of therapeutic molecules to said body prior to, while or after applying through said electrodes 60 one or more electric pulses to create a transient permeabilization of cell membranes of tissue in said target region.

In a variation of this method said dose of therapeutic molecules may be administered systemically.

In an alternative variation of this method said dose may be administered locally in the vicinity of the target region.

In a variation of the latter variation said dose may be delivered before, during or after extending said electrodes 60, through a delivery channel 20 extending through the length of said shaft 10 and terminating through said distal tip 13.

The Electroporation device according to the invention and as described above may in various embodiments further be applied for a method A of generating an electric field in a target region of a patient, comprising the steps of
- inserting into the vicinity of the target region a set of electrodes 60, having respective distal ends 61, enclosed within a single elongate introducer shaft 10 having a distal tip 13 formed with a substantially smooth, rounded, non-cutting shape with a substantially smooth, non-cutting transition to the introducer shaft 10;
- extending at least a pair of said electrodes 60 to an extended position, such that the electrode distal ends 61 are extended to a position distally of said distal tip 13, and such that said electrode distal ends 61 are deflected away from a longitudinal axis L of said shaft 10 in such a way that at least one planar projection taken in a plane perpendicular to said longitudinal axis L of a distance between a pair of distal ends 61 of said electrodes 60 is larger than a maximal extent of a cross-section of said introducer shaft 10, said cross-section taken in a plane perpendicular to said a longitudinal axis L at a distal end 11 of said introducer shaft 10, the deflection of said distale ends 61 of said electrodes 60 being provided by a curving of distributor channels 70 formed in said distal tip; and
- applying through said electrodes 60 one or more electric pulses to the target tissue to create a transient permeabilization of cell membranes of tissue in said target region.

The Electroporation device according to the invention and as described above may in various embodiments further be applied for a method B for generating an electric field in a target tissue of a patient, comprising the steps of
- inserting into the vicinity of a target tissue a set of point electrodes 60, having respective electrically conductive distal ends 61, and positioning said electrode distal ends 61 in a spatial formation surrounding or enclosing at least partly said target tissue;
- applying through said point electrodes 60 one or more electric pulses to the target tissue to create a transient permeabilization of cell membranes of tissue in said target region.

In a variation of the latter method the set of electrodes are inserted via a single elongate introducer shaft 10 having a distal tip 13 formed with a substantially smooth, rounded, non-cutting shape with a substantially smooth, non-cutting transition to the introducer shaft 10, and where the set of electrodes 60 are extended to an extended position, such that the electrode distal ends 61 are extended to a position distally of said distal tip 13, and such that said electrode distal ends 61 are deflected away from a longitudinal axis L of said shaft 10 in such a way that at least one planar projection taken in a plane perpendicular to said longitudinal axis L of a distance between a pair of distal ends 61 of said electrodes 60 is larger than a maximal extent of a cross-section of said introducer shaft 10, said cross-section taken in a plane perpendicular to said a longitudinal axis L at a distal end 11 of said introducer shaft 10, the deflection of said distale ends 61 of said electrodes 60 being provided by a curving of distributor channels 70 formed in said distal tip;

In a variation of this method the point electrodes may be positioned such that when a sequence of electric pulses is applied through said electrodes an ellipsoid or spatially ellipsoid electric field is generated in the tissue.

In a variation of this method said ellipsoid or spatial ellipsoid field may be generated by positioning said point electrodes in an ellipsoid or spatially ellipsoid configuration at least partly surrounding or enclosing said target tissue.

In a variation of this method said point electrode distal ends 61 may be positioned in substantially circular parallel layers and where the position of the point electrodes in a section perpendicular to said circular layers defines an ellipsoid configuration.

In a variation of the latter variation, the electric field may be generated in the tissue by applying a sequence of electric pulses between at least sixteen point electrodes 61 in at least four essentially parallel, consecutive layers a, b, c, d, comprising at least four point electrodes 61 in each layer a, b, c, d, and wherein said sequence comprises the steps of generating at least some pulses travelling from a first positive layer a of point electrodes to a first negative layer of point electrodes c placed in equidistant relation to the electrodes in the first layer a, while other pulses simultaneously travel from a second positive layer b to a second negative layer of point electrodes d, respectively.

## Claims

1. An electroporation device (1) comprising
- a handle section (100);
- an elongate introducer shaft (10) connected to said handle section (100), said introducer shaft (10) having a distal tip (13); and
- a set of electrodes (60) having respective distal ends (61), each electrode (60) being slidably arranged within said introducer shaft (10) and said tip (13) from a retracted position, where said distal ends (61) are enclosed within said introducer shaft (10), to an exposed position, where said distal ends (61) extend from said distal tip (13);
wherein said electrode distal ends (61) are deflectable away from a longitudinal axis (L) of said shaft (10) when deployed/extended to their extended position, such that at least one planar projection taken in a plane perpendicular to said longitudinal axis (L) of a distance (D1) between a pair of distal ends (61) of said electrodes (60) is larger than a maximal extent (D2) of a cross-section of said introducer shaft (10), said cross-section taken in a plane perpendicular to said longitudinal axis (L) at a distal end (11) of said introducer shaft (10),
wherein the deflection of said distal ends (61) of said electrodes (60), when in their extended position, is provided by a curving of distributor channels (70) formed in said distal tip (13),
wherein the distal tip (13) is formed with a substantially smooth, rounded, non-cutting shape with a substantially smooth, non-cutting transition to the introducer shaft (10), and
wherein the electrode distal ends (61) are extendable to a position distally of said distal tip (13), and in a strictly linear path.

2. An electroporation device (1) according to claim 1 comprising ten or more electrodes (60).

3. An electroporation device (1) according to claim 1 or 2, wherein an electrical pulse can be fired from one electrode (60) to another electrode (60) of the device (1).

4. An electroporation device (1) according to any of claims 1-3, wherein said introducer shaft (10) further comprises a delivery channel (20) through which a dose of therapeutical molecules can be administered, said delivery channel (20) extending through the length of said shaft (10) and terminating through said distal tip (13).

5. An electroporation device (1) according to any of claims 1-4 wherein the distal tip (13) is detachable from said introducer shaft (10).

6. An electroporation device (1) according to any of claims 1-5, wherein each of said electrodes (60) can be advanced individually or in sets to their extended positions.

7. An electroporation device (1) according to any of claims 1-6, wherein said electrodes (60) are extendable such that their distal ends (61) form a spatial distribution around a volume of target tissue.

8. An electroporation device (1) according to claim 7, wherein said electrodes (60) are extendable such that their distal ends (61) form a substantially spherical distribution pattern.

9. An electroporation device (1) according to claim 7, wherein a subset of said electrodes (60) are extendable, such that their distal ends (61) form an ellipsoid pattern (E) in a plane parallel to said longitudinal axis (L) when extended.

10. An electroporation device (1) according to any of claims 1-9, wherein said electrodes (60) are slideably arranged in electrically insulated guide channels (50).

11. An electroporation device (1) according to any of claims 1-9, wherein said electrodes (60) are provided with an electric insulation coating, the distal-most part of the electrode (60) distal ends (61) being un-insulated to form point electrodes.

12. An electroporation device (1) according to any of claims 4-11, having a central delivery channel (20), that is connectable to an external therapeutic molecule delivery system (115) comprising a therapeutic molecule reservoir and pumping means for administering said therapeutic molecules through said delivery channel (20).

13. An electroporation device (1) according to any of claims 4-11, wherein the handle part (100) comprises a therapeutic molecule delivery system comprising a therapeutic molecule reservoir and actuating means for administering said therapeutic molecules through said delivery channel (20).

14. An electroporation device (1) according to any one of claims 12-13, wherein said device (1) is further adapted to for introducing a surgical tool or an ultrasound probe through said delivery channel (20).

15. An electroporation device (1) according to any of the previous claims, wherein said introducer shaft (10) has a circular cross section with an outer diameter (D2) of 15 mm or less, preferably of 10 mm or less, more preferably of 5 mm or less.

16. An electroporation device (1) according to any of the previous claims, wherein the introducer shaft (10) comprises an outer tube (15) and an inner electrode assembly guide (16) received in said outer tube (15), and where said electrodes (60) are slideably arranged in electrode guide channels (50) formed in said inner electrode assembly guide (16).

17. An electroporation device (1) according to claim 16, wherein said electrode guide channels (50) are formed in a set of cylindrical guide sheaths (30) that are received in longitudinal semi-open channels (17) distributed radially along the periphery of said inner electrode assembly guide (16).

18. An electroporation device (1) according to any of the previous claims comprising 32 electrodes (60).

19. An electroporation device (1) according to claim 18, wherein said electrodes (60) are slideably arranged within guide channels (50) distributed in groups of four in each of eight cylindrical guide sheaths (30).

20. An electroporation device (1) according to any of the previous claims, wherein an electric stimulus generator is integrated into the handle section (100) of the device (1).

21. An electroporation device (1) according to any of the claims 1-19, having means for attaching the device (1) electrodes (60) to an external electric stimulus generator.

22. An electroporation device (1) according to claim 20 or 21, wherein each electrode (60) is individually assignable, such that the emission of electric stimuli can be provided from individual electrodes (60).

23. An electroporation system, said system comprising an electroporation device (1) according to any one of claims 1-22, and an electric stimulus generator, wherein said system is adapted to provide an electrical field in a target tissue, by applying an series of electrical pulses between electrodes (60) of said device such that a transient permeabilization of cell membranes of cells in a target tissue is provided.

## Patentansprüche

1. Elektroporationsvorrichtung (1), umfassend
- einen Halteabschnitt (100);
- einen länglichen Einführungsschaft (10), der mit dem Halteabschnitt (100) verbunden ist, wobei der Einführungsschaft (10) eine distale Spitze (13) aufweist; und
- einen Satz Elektroden (60) mit jeweils einem distalen Ende (61), wobei jede Elektrode (60) in dem Einführungsschaft (10) und der Spitze (13) von einer zurückgezogenen Position, in der die distalen Enden (61) vom Einführungsschaft (10) umgeben sind, in eine freiliegende Position, in der sich die distalen Enden (61) aus der distalen Spitze (13) erstrecken, gleitend angeordnet ist;
wobei die distalen Elektrodenenden (61) beim Einsetzen/Ausziehen in ihre ausgezogene Position von einer Längsachse (L) des Schafts (10) ablenkbar sind,
sodass mindestens eine planare Projektion in einer Ebene rechtwinklig zur Längsachse (L) eines Abstands (D1) zwischen einem Paar distaler Enden (61) der Elektroden (60) größer ist als eine maximale Ausdehnung (D2) eines Querschnitts des Einführungsschafts (10), wobei der Querschnitt in einer Ebene senkrecht zur Längsachse (L) an einem distalen Ende (11) des Einführungsschafts (10) liegt,
wobei die Ablenkung des distalen Endes (61) der Elektroden (60) in ausgezogener Position durch die Krümmung von Verteilerkanälen (70) bereitgestellt wird, die in der distalen Spitze (13) ausgebildet sind,
wobei die distale Spitze (13) mit einer im Wesentlichen glatten, gerundeten, nicht schneidenden Form mit einem im Wesentlichen glatten, nicht schneidenden Übergang zum Einführungsschaft (10) ausgebildet ist, und
wobei die distalen Elektrodenenden (61) in einem streng linearen Weg in eine Position ausziehbar sind, die sich distal zur distalen Spitze (13) befindet.

2. Elektroporationsvorrichtung (1) nach Anspruch 1, umfassend zehn oder mehr Elektroden (60).

3. Elektroporationsvorrichtung (1) nach Anspruch 1 oder 2, wobei ein elektrischer Impuls von einer Elektrode (60) an eine andere Elektrode (60) der Vorrichtung (1) abgegeben werden kann.

4. Elektroporationsvorrichtung (1) nach einem der Ansprüche 1-3, wobei der Einführungsschaft (10) weiterhin einen Abgabekanal (20) umfasst, durch den eine Dosis therapeutischer Moleküle verabreicht werden kann, wobei sich der Abgabekanal (20) über die Länge des Schafts (10) erstreckt und durch die distale Spitze (13) endet.

5. Elektroporationsvorrichtung (1) nach einem der Ansprüche 1-4, wobei die distale Spitze (13) von dem Einführungsschaft (10) abnehmbar ist.

6. Elektroporationsvorrichtung (1) nach einem der Ansprüche 1-5, wobei jede der Elektroden (60) einzeln oder in Sätzen in ihrer ausgefahrenen Position vorgeschoben werden kann.

7. Elektroporationsvorrichtung (1) nach einem der Ansprüche 1-6, wobei die Elektroden (60) derart ausziehbar sind, dass ihr distales Ende (61) eine räumliche Anordnung um ein Volumen des Zielgewebes bildet.

8. Elektroporationsvorrichtung (1) nach Anspruch 7, wobei die Elektroden (60) derart ausziehbar sind, dass ihr distales Ende (61) ein im Wesentlichen kugelförmiges Verteilungsmuster ausbildet.

9. Elektroporationsvorrichtung (1) nach Anspruch 7, wobei eine Untergruppe der Elektroden (60) derart ausziehbar ist, dass ihr distales Ende (61) im ausgezogenen Zustand ein ellipsenförmiges Muster (E) in einer Ebene parallel zur Längsachse (L) ausbildet.

10. Elektroporationsvorrichtung (1) nach einem der Ansprüche 1-9, wobei die Elektroden (60) gleitend in elektrisch isolierten Führungskanälen (50) angeordnet sind.

11. Elektroporationsvorrichtung (1) nach einem der Ansprüche 1-9, wobei die Elektroden (60) mit einem elektrisch isolierenden Überzug versehen sind, wobei der distalste Teil des distalen Endes (61) der Elektrode (60) unter Ausbildung einer punktförmigen Elektrode abisoliert ist.

12. Elektroporationsvorrichtung (1) nach einem der Ansprüche 4-11 mit einem mittigen Abgabekanal (20), der an ein externes Abgabesystem für therapeutische Moleküle (115), umfassend einen Behälter für therapeutische Moleküle und Pumpmittel zur Verabreichung der therapeutischen Moleküle durch den Abgabekanal (20), anschließbar ist.

13. Elektroporationsvorrichtung (1) nach einem der Ansprüche 4-11, wobei der Halteabschnitt (100) ein Abgabesystem für therapeutische Moleküle umfasst, umfassend einen Behälter für therapeutische Moleküle und Pumpmittel zur Verabreichung der therapeutischen Moleküle durch den Abgabekanal (20).

14. Elektroporationsvorrichtung (1) nach einem der Ansprüche 12-13, wobei die Vorrichtung (1) weiterhin zur Einführung eines chirurgischen Instruments oder einer Ultraschallsonde durch den Abgabekanal (20) beschaffen ist.

15. Elektroporationsvorrichtung (1) nach einem der vorhergehenden Ansprüche, wobei der Einführungsschaft (10) einen kreisförmigen Querschnitt mit einem Außendurchmesser (D2) von 15 mm oder weniger, vorzugsweise 10 mm oder weniger, mehr bevorzugt 5 mm oder weniger, aufweist.

16. Elektroporationsvorrichtung (1) nach einem der vorhergehenden Ansprüche, wobei der Einführungsschaft (10) ein Außenrohr (15) und eine innere Elektrodengruppenführung (16), die in dem Außenrohr (15) aufgenommen ist, umfasst und wobei die Elektroden (60) in Elektrodenführungskanälen (50), die in der inneren Elektrodengruppenführung (16) ausgebildet sind, gleitend angeordnet sind.

17. Elektroporationsvorrichtung (1) nach Anspruch 16, wobei die Elektrodenführungskanäle (50) in einem Satz zylindrischer Führungsscheiden (30) ausgebildet sind, die in länglichen, halb offenen Kanälen (17) aufgenommen sind, welche radial am Umfang der inneren Elektrodengruppenführung (16) verteilt sind.

18. Elektroporationsvorrichtung (1) nach einem der vorhergehenden Ansprüche, umfassend 32 Elektroden (60).

19. Elektroporationsvorrichtung (1) nach Anspruch 18, wobei die Elektroden (60) in Führungskanälen (50), die im Gruppen zu je vier in jedem der acht zylindrischen Führungsscheiden (30) verteilt sind, gleitend angeordnet sind.

20. Elektroporationsvorrichtung (1) nach einem der vorhergehenden Ansprüche, wobei ein Generator elektrischer Impulse in den Halterabschnitt (100) der Vorrichtung (1) integriert ist.

21. Elektroporationsvorrichtung (1) nach einem der Ansprüche 1-19 mit Mitteln zum Befestigen der Elektroden (60) der Vorrichtung (1) an einem externen Generator elektrischer Impulse.

22. Elektroporationsvorrichtung (1) nach einem der Ansprüche 20 oder 21, wobei jede Elektrode (60) einzeln zuweisbar ist, sodass die Abgabe elektrischer Reize von einzelnen Elektroden (60) bereitgestellt werden kann.

23. Elektroporationssystem, wobei das System eine Elektroporationsvorrichtung (1) nach einem der Ansprüche 1-22 und einen Generator elektrischer Reize umfasst, wobei das System derart beschaffen ist, dass durch Aussenden einer Serie elektrischer Impulse zwischen den Elektroden (60) der Vorrichtung in einem Zielgewebe ein elektrisches Feld bereitgestellt wird, sodass eine vorübergehende Permeabilisierung der Zellmembranen der Zellen in einem Zielgewebe bereitgestellt ist.

## Revendications

1. Dispositif d'électroporation (1) comprenant
- une partie poignée (100) ;
- une tige d'introduction allongée (10) raccordée à ladite partie poignée (100), ladite tige d'introduction (10) possédant une pointe distale (13) ; et
- un ensemble d'électrodes (60) possédant des extrémités distales respectives (61), chaque électrode (60) étant disposée de manière à pouvoir coulisser à l'intérieur de ladite tige d'introduction (10) et ladite pointe (13) depuis une position rentrée, dans laquelle lesdites extrémités distales (61) sont enfermées dans ladite tige d'introduction (10), vers une position exposée, dans laquelle lesdites extrémités distales (61) sortent de ladite pointe distale (13) ;
dans lequel lesdites extrémités distales des électrodes (61) peuvent être déviées par rapport à un axe longitudinal (L) de ladite tige (10) quand elles sont déployées/sorties en position externe, de manière à ce qu'au moins une projection planaire dans un plan perpendiculaire au dit axe longitudinal (L) d'une distance (D1) entre une paire d'extrémités distales (61) desdites électrodes (60) soit supérieure à une dimension maximale (D2) d'une section de ladite tige d'introduction (10), ladite section étant prise dans un plan perpendiculaire au dit axe longitudinal (L) au niveau d'une extrémité distale (11) de ladite tige d'introduction (10),
dans lequel la déviation desdites extrémités distales (61) desdites électrodes (60), dans leur position sortie, est assurée par une courbure de canaux de distribution (70) formés dans ladite pointe distale (13),
dans lequel la pointe distale (13) présente une forme essentiellement lisse, arrondie, non coupante avec une transition essentiellement lisse, non coupante vers la tige d'introduction (10), et
dans lequel les extrémités distales des électrodes (61) peuvent s'étendre vers une position opposée à ladite pointe distale (13), et selon une trajectoire strictement linéaire.

2. Dispositif d'électroporation (1) selon la revendication 1 comprenant au moins dix électrodes (60).

3. Dispositif d'électroporation (1) selon les revendications 1 ou 2, dans lequel une impulsion électrique peut être émise d'une électrode (60) vers une autre électrode (60) du dispositif (1).

4. Dispositif d'électroporation (1) selon l'une quelconque des revendications 1 à 3, dans lequel ladite tige d'introduction (10) comprend également un canal d'administration (20) au travers duquel une dose de molécules thérapeutiques peut être administrée, ledit canal d'administration (20) passant dans toute la longueur de ladite tige (10) et se terminant au travers de ladite pointe distale (13).

5. Dispositif d'électroporation (1) selon l'une quelconque des revendications 1 à 4, dans lequel la pointe distale (13) peut être détachée de ladite tige d'introduction (10).

6. Dispositif d'électroporation (1) selon l'une quelconque des revendications 1 à 5, dans lequel chacune desdites électrodes (60) peut être avancée individuellement ou en groupe vers sa position sortie.

7. Dispositif d'électroporation (1) selon l'une quelconque des revendications 1 à 6, dans lequel lesdites électrodes (60) peuvent s'étendre de manière à ce que leurs extrémités distales (61) soient réparties dans l'espace autour d'un volume de tissu cible.

8. Dispositif d'électroporation (1) selon la revendication 7, dans lequel lesdites électrodes (60) peuvent s'étendre de manière à ce que leurs extrémités distales (61) soient réparties selon un motif essentiellement sphérique.

9. Dispositif d'électroporation (1) selon la revendication 7, dans lequel un sous-ensemble desdites électrodes (60) peut s'étendre de manière à ce que leurs extrémités distales (61) forment un motif ellipsoïde (E) dans un plan parallèle au dit axe longitudinal (L) lorsqu'elles sont sorties.

10. Dispositif d'électroporation (1) selon l'une quelconque des revendications 1 à 9, dans lequel lesdites électrodes (60) sont disposées de manière à pouvoir coulisser dans des canaux de guidage (50) électriquement isolés.

11. Dispositif d'électroporation (1) selon l'une quelconque des revendications 1 à 9, dans lequel lesdites électrodes (60) sont dotées d'un revêtement électriquement isolant, la partie la plus distale des extrémités distales (61) des électrodes (60) n'étant pas isolée afin de former des électrodes ponctuelles.

12. Dispositif d'électroporation (1) selon l'une quelconque des revendications 4 à 11, possédant un canal d'administration central (20) pouvant être raccordé à un système d'administration de molécules thérapeutiques externe (115) comprenant un réservoir de molécules thérapeutiques et un moyen de pompage pour administrer lesdites molécules thérapeutiques au travers dudit canal d'administration (20).

13. Dispositif d'électroporation (1) selon l'une quelconque des revendications 4 à 11, dans lequel la partie poignée (100) comprend un système d'administration de molécules thérapeutiques comprenant un réservoir de molécules thérapeutiques et un moyen de commande pour administrer lesdites molécules thérapeutiques au travers dudit canal d'administration (20).

14. Dispositif d'électroporation (1) selon l'une quelconque des revendications 12 à 13, dans lequel ledit dispositif (1) est également adapté à l'introduction d'un instrument chirurgical ou d'une sonde à ultrasons au travers dudit canal d'administration (20).

15. Dispositif d'électroporation (1) selon l'une quelconque des revendications précédentes, dans lequel ladite tige d'introduction (10) possède une section circulaire de diamètre extérieur (D2) inférieur ou égal à 15 mm, préférentiellement inférieur ou égal à 10 mm, plus préférentiellement inférieur ou égal à 5 mm.

16. Dispositif d'électroporation (1) selon l'une quelconque des revendications précédentes, dans lequel la tige d'introduction (10) comprend un tube externe (15) et un guide interne de montage d'électrodes (16) logé dans ledit tube externe (15), et où lesdites électrodes (60) sont disposées de manière à pouvoir coulisser dans des canaux de guidage d'électrodes (50) formés dans ledit guide interne de montage d'électrodes (16).

17. Dispositif d'électroporation (1) selon la revendication 16, dans lequel lesdits canaux de guidage d'électrodes (50) sont formés dans un ensemble de gaines de guidage cylindriques (30) qui sont logées dans des canaux semi-ouverts longitudinaux (17) répartis radialement le long de la périphérie dudit guide interne de montage d'électrodes (16).

18. Dispositif d'électroporation (1) selon l'une quelconque des revendications précédentes comprenant 32 électrodes (60).

19. Dispositif d'électroporation (1) selon la revendication 18, dans lequel lesdites électrodes (60) sont disposées de manière à pouvoir coulisser dans des canaux de guidage (50) répartis par groupes de quatre dans chacune des huit gaines de guidage cylindriques (30).

20. Dispositif d'électroporation (1) selon l'une quelconque des revendications précédentes, dans lequel un générateur de stimuli électriques est intégré à la partie poignée (100) du dispositif (1).

21. Dispositif d'électroporation (1) selon l'une quelconque des revendications 1 à 19, possédant un moyen pour relier les électrodes (60) du dispositif (1) à un générateur de stimuli électriques.

22. Dispositif d'électroporation (1) selon les revendications 20 ou 21, dans lequel chaque électrode (60) peut être affectée de façon individuelle, de manière à ce que l'émission de stimuli électriques puisse se faire depuis des électrodes individuelles (60).

23. Système d'électroporation, ledit système comprenant un dispositif d'électroporation (1) selon l'une quelconque des revendications 1 à 22, et un générateur de stimuli électriques, dans lequel ledit système est adapté à la création d'un champ électrique dans un tissu cible, en appliquant une série d'impulsions électriques entre les électrodes (60) dudit dispositif de manière à induire une perméabilisation transitoire des membranes cellulaires des cellules d'un tissu cible.
